# EUROPEAN PATENT APPLICATION

(11) **EP 2 647 627 A1**
(43) Date of publication of application: **09.10.2013**
(21) Application number: 12382134.0
(22) Date of filing: 02.04.2012
(51) Int. Cl.: C07D 215/22, A61K 31/4704

(54) **Salts of 5-[(1r)-2-({2-[4-(2,2-difluoro-2-phenylethoxy)phenyl] ethyl}amino)-1-hydroxyethyl]-8-hydroxyquinolin-2(1h)-one.**

(71) Applicant: Almirall, S.A., 08022 Barcelona (ES)
(72) Inventor: Puig Duran, Carlos, 08980 Sant Feliu de Llobregat (ES); Carrera Carrera, Francesc, 08980 Sant Feliu de Llobregat (ES); Pérez Garcia, Juan Bautista, 08980 Sant Feliu de Llobregat (ES); Moyes Valls, Enrique, 08980 Sant Feliu de Llobregat (ES); Marchueta Hereu, Iolanda, 08980 Sant Feliu de Llobregat (ES)
(74) Representative: Elzaburu Marquez, Alberto

(57) **Abstract**

The present invention is directed to a pharmaceutically acceptable crystalline addition salt of (i) 5-[(1R)-2-({2-[4-(2,2-difluoro-2-phenylethoxy)phenyl]ethyl}amino)-1-hydroxyethyl]-8-hydroxyquinolin-2(1H)-one, and (ii) a dicarboxylic acid, a sulfonic acid or a sulfimide, or a pharmaceutically acceptable solvate thereof.

## Description

### Field of the invention

The present invention is directed to novel crystalline salts of 5-[(1R)-2-({2-[4-(2,2-difluoro-2-phenylethoxy)phenyl]ethyl}amino)-1-hydroxyethyl]-8-hydroxyquinolin-2(1H)-one, and solvates thereof. The invention is also directed to pharmaceutical compositions comprising the salts, methods of using them to treat respiratory diseases associated with β2 adrenergic receptor activity, and processes and intermediates useful for preparing such salts.

### Background of the invention

Many compounds are known in the art with activity as β2 adrenergic receptor agonists. Of the various β2-agonist compounds known, many cannot be formulated for effective delivery by inhalation as a dry powder. Delivery by inhalation as a dry powder is challenging. It requires careful control of the particle size of the powder which is to be inhaled, and careful control of the particle size distribution. Further, it is important to avoid particle agglomeration or aggregation.

It has now been found that certain salts of the β2-agonist compound 5-[(1R.)-2-({2-[4-(2,2-difluoro-2-phenylethoxy)phenyl]ethyl}amino)-1-hydroxyethyl]-8-hydroxyquinolin-2(1H)-one form stable crystal forms, which can be formulated into effective dry powders of administration by inhalation. 5-[(1R)-2-({2-[4-(2,2-difluoro-2-phenylethoxy)phenyl] ethyl}amino)-1-hydroxyethyl]-8-hydroxyquinolin-2(1H)-one is described in published patent application WO 2008/046598 A1.

### Summary of the invention

The present invention provides a pharmaceutically acceptable crystalline addition salt of (i) 5-[(1R)-2-({2-[4-(2,2-diHuoro-2-phenylethoxy)phenyl]ethyl}amino)-1-hydroxyethyl]-8-hydroxyquinolin-2(1H)-one, and (ii) a dicarboxylic acid, a sulfonic acid or a sulfimide, or a pharmaceutically acceptable solvate thereof.

The invention also provides a pharmaceutical composition comprising a therapeutically effective amount of a salt of the invention and a pharmaceutically= acceptable carrier.

The invention further provides a combination comprising a salt of the invention and one or more other therapeutic agents.

The invention also provides a salt of the invention, a pharmaceutical composition of the invention or a combination of the invention, for use in the treatment of a pathological condition or disease associated with β2 adrenergic receptor activity.

The invention further provides use of a salt of the invention, a pharmaceutical composition of the invention or a combination of the invention, in the manufacture of a medicament for the treatment of a pathological condition or disease associated with β2 adrenergic receptor activity.

The invention also provides a method for treating a subject afflicted with a pathological condition or disease associated with β2 adrenergic receptor activity, which comprises administering to said subject an effective amount of a salt of the invention, a pharmaceutical composition as of the invention or a combination of the invention.

### Brief description of figures

Figure 1 shows the ¹H-NNIR (400 MHz, *d4*-methanal) for the 5-[(1R)-2-({2-[4-(2,2-dif)uoro-2-phenylethoxy)phenyl]ethyl}amino)-1-hydroxyethyl]-8-hydroxyquinolin-2(1H)-one.
Figure 2 shows the powder X-ray diffraction (PXRD) pattern for 5-[(1R)-2-({2-[4-(2,2-difluoro-2-phenylethoxy)phenyl]ethyl}amino)-1-hydroxyethyl]-8-hydroxyquinolin-2(IH)-one fumarate.
Figure 3 shows the ¹H-NMR (400 MHz, *d4*-methanol) for 5-[(1R)-2-({2-[4-(2,2-difluoro-2-phenylethoxy)phenyl]ethyl}amino)-1-hydroxyethyl]-8-hydroxyquinolin-2(1H)-one fumarate.
Figure 4 shows the differential scanning calorimetry (DSC) analysis of 5-[(1R)-2-({2-[4-(2,2-difluoro-2-phenylethoxy)phenyl]ethyl}amino)-1-hydroxyethyl]-8-hydroxyquinolin-2(1H)-one fumarate.
Figure 5 shows the thermogravimetric (TG) analysis of5-[(1R)-2-({2-[4-(2,2-difluoro-2-phenylethaxy)phenyl]ethyl}amino)-1-hydraxyethyl]-8-hydraxy=quinolin-2(1H)-one fumarate.
Figure 6 shows the Fourier transform infrared (FTIR) spectrum for 5-[(1R)-2-({2-[4-(2,2-diSuoro-2-phenylethoxy)phenyl]ethyl) amino)-1 -hydroxyethyl]-8-hydroxyquinolin-2(IH)-one fumarate.
Figure 7 shows the PXRD analysis of 5-[(1R)-2-({2-[4-(2,2-difluoro-2-phenylethoxy)phenyl]ethyl}amino)-1-hydroxyethyl]-8-hydroxyquinolin-2(1H)-one succinate.
Figure 8 is the ¹H-NMR spectrum of 5-[(1R)-2-({2-[4-(2,2-difluoro-2-phenylethoxy)phenyl]ethyl}amino)-1-hydroxyethyl]-8-hydroxyquinolin-2(1H)-one succinate.
Figure 9 shows the DSC analysis of 5-[(1R)-2-({2-[4-(2,2-difluoro-2-phenylethoxy)phenyl]ethyl}amino)-1-hydroxyethyl]-8-hydroxyquinolin-2(1H)-one succinate.
Figure 10 shows the TG analysis of 5-[(1R)-2-({2-[4-(2,2-difluoro-2-phenylethoxy)phenyl]ethyl}amino)-1-hydroxyethyl]-8-hydroxyquinalin-2(1H)-one succinate.
Figure 11 shows the FTIR spectrum for 5-[(1R)-2-({2-[4-(2,2-difluoro-2-phenylethoxy)phenyl]ethyl}amino)-1-hydroxyethyl]-8-hydroxyquinolin-2(1H)-one succinate.
Figures 12 and 13 show the PXRD analysis of 5-[(1R)-2-({2-[4-(2,2-difluoro-2-phenylethoxy)phenyl]ethyl}amino)-1-hydroxyethyl]-8-hydroxyquinolin-2(1H)-one methanesulfonate. Figure 13 is an enlargement of a portion of Figure 12.
Figure 14 is the ¹H-NMR spectrum of 5-[(1R)-2-({2-[4-(2,2-difluoro-2-phenylethoxy)phenyl]ethyl}amino)-1-hydroxyethyl]-8-hydroxyquinalin-2(1H)-one methanesulfonate.
Figure 15 shows the DSC analysis of 5-[(1R)-2-({2-[4-(2,2-difluoro-2-phenylethoxy)phenyl]ethyl}amino)-1-hydroxyethyl]-8-hydroxyquinolin-2(1H)-one methanesulfonate.
Figure 16 shows further DSC analysis on the 5-[(1R)-2-({2-[4-(2,2-difluoro-2-phenylethoxy)phenyl]ethyl}amino)-1-hydroxyethyl]-8-hydroxyquinolin-2(1H)-one methanesulfonate.
Figure 17 shows the TG analysis of 5-[(1R)-2-({2-[4-(2,2-difluoro-2-phenylethoxy)phenyl]ethyl}amino)-1-hydroxyethyl]-8-hydroxyquinolin-2(1H)-one methanesulfonate.
Figure 18 shows the FTIR spectrum for 5-[(1R)-2-({2-[4-(2,2-difluoro-2-phenylethoxy)phenyl]ethyl}amino)-1-hydroxyethyl]-8-hydroxyquinalin-2(1H)-one methanesulfonate.
Figure 19 shows the PXRD analysis of 5-[(1R)-2-({2-[4-{2,2-difluoro-2-phenylethaxy)phenyl]ethyl}amino)-1-hydroxyethyl]-8-hydroxyquinalin-2(1H)-one saccharinate hemihydrate.
Figure 20 is the ¹H-N-N4R spectrum of 5-[(1R)-2-({2-[4-(2,2-difluoro-2-phenylethoxy)phenyl]ethyl}amino)-1-hydroxyethyl]-8-hydroxyquinolin-2(1H)-one saccharinate hemihydrate.
Figure 21 shows the DSC analysis of 5-[(1R)-2-({2-[4-(2,2-difluoro-2-phenylethoxy)phenyl]ethyl}amino)-1-hydroxyethyl]-8-hydroxyquinolin-2(1H)-one saccharinate hemihydrate.
Figure 22 shows the TG analysis of 5-[(1R)-2-({2-[4-(2,2-difluoro-2-phenylethoxy)phenyl]ethyl}amino)-1-hydroxyethyl]-8-hydroxyquinolin-2(1H)-one saccharinate hemihydrate.
Figure 23 shows the FTIR spectrum for 5-[(1R)-2-({2-[4-(2,2-diftuoro-2-phenylethoxy)phenyl]ethyl}amino)-1-hydroxyethyl]-8-hydroxyquinolin-2(1H)-one saccharinate hemihydrate.

### Detailed description of the invention

When describing the salts, compositions and methods of the invention, the following terms have the following meanings, unless otherwise indicated.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "condition or disease associated with β2 adrenergic receptor activity" includes all disease states and/or conditions that are acknowledged now, or that are found in the future, to be associated with β2 adrenergic receptor activity. Such disease states include, but are not limited to asthma and chronic obstructive pulmonary disease (including chronic bronchitis and emphysema).

The term "solvate" refers to a complex or aggregate formed by one or more molecules of a solute, i.e. a salt of the invention or a pharmaceutically-acceptable salt thereof, and one or more molecules of a solvent. Such solvates are typically crystalline solids having a substantially fixed molar ratio of solute and solvent. Representative solvents include by way of example, water, ethanol, isopropanol and the like. The preferred solvate is a hydrate, for example a hemihydrate.

### Pharmaceutically acceptable crystalline salts

The pharmaceutically acceptable crystalline salt of the invention is an addition salt of (i) 5-[(1R)-2-({2-[4-(2,2-difluoro-2-phenylethoxy)phenyl]ethyl}amino)-1-hydraxyethyl]-8-hydraxyquinalin-2(1H)-one, and (ii) a dicarboxylic acid, a sulfonic acid or a sulfimide, or a pharmaceutically acceptable solvate thereof.

5-[(1R)-2-({2-[4-(2,2-difluoro-2-phenylethoxy)phenyl]ethyl}amino)-1-hydroxyethyl]-8-hydroxyquinalin-2(1H)-one has the following structure:

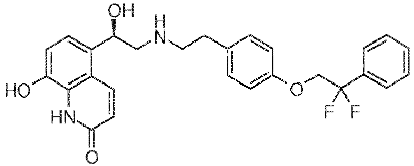

Typically, the salt is an addition salt of (ii) a dicarboxylic acid. The dicarboxylic acid is preferably fumaric acid or succinic acid. Fumaric acid is particularly preferred. Thus, preferred salts of the invention are: 5-[(1R)-2-({2-[4-(2,2-difluoro-2-phenylethaxy)phenyl]ethyl}amino)-1-hydraxyethyl]-8-hydraxyquinalin-2(1H)-one fumarate and 5-[(1R)-2-({2-[4-{2,2-difluara-2-phenylethaxy)phenyl]ethyl}amino)-1-hydroxyethyl]-8-hydroxyquinotin-2(1H)-one succinate, and pharmaceutically acceptable solvates thereof. Particularly preferred is 5-[(1R)-2-({2-[4-(2,2-difluoro-2-phenylethoxy)phenyl]ethyl}amino)-1-hydroxyethyl]-8-hydroxyquinolin-2(1H)-one fumarate and pharmaceutically acceptable solvates thereof.

Alternatively, the salt is an addition salt of(ii) a sulfonic acid. The sulfonic acid is preferably methanesulfonic acid. Thus, a further preferred salt of the invention is 5-[(1R)-2-({2-[4-(2,2-difluoro-2-phenylethoxy)phenyl]ethyl}amino)-1-hydroxyethyl]-8-hydraxyquinolin-2(1H)-one methanesulphonate, or a pharmaceutically acceptable solvate thereof.

Alternatively, the salt is an addition salt of(ii) a sulfimide. The sulfimide is preferably saccharin. Thus, a further preferred salt of the invention is 5-[(1R)-2-({2-[4-(2,2-difluoro-2-phenylethoxy)phenyl]ethyl}amino)-1-hydroxyethyl]-8-hydroxyquinolin-2(1H)-one saccharinate, or a pharmaceutically acceptable solvate thereof. Preferred solvate forms are 5-[(1R)-2-({2-[4-(2,2-difluoro-2-phenylethoxy)phenyl]ethyl}amino)-1-hydroxyethyl]-8-hydroxyquinolin-2(1H)-one saccharinate hemimethanolate and 5-[(1R)-2-({2-[4-(2,2-difluoro-2-phenylethoxy)phenyl]ethyl}amino)-1-hydroxyethyl]-8-hydroxvquinolin-2(1H)-one saccharinate hemihydate. The hemihydrate is particularly preferred.

### General synthetic procedures

The salts of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Processes for preparing salts of the invention are provided as further embodiments of the invention and are illustrated by the procedures below.

The salts of the invention can be synthesized from 5-[(1R)-2-({2-[4-(2,2-difluoro-2-phenylethoxy)phenyl]ethyl}amino)-1-hydroxyethyl]-8-hydroxyquinalin-2(1H)-one and from the appropriate dicarboxylic acid, sulfonic acid or sulfimide, which will generally be commercially available from, for example, Aldrich. For example:
- the fumarate salt is typically prepared from fumaric acid;
- the succinate salt is typically prepared from succinic acid;
- the methane sulfonate salt is typically prepared from methanesulfonic acid; and
- the saccharinate salt is typically prepared from saccharin.

Suitable solvents for this reaction can be selected by a skilled chemist and may depend on the specific salt to be formed. Mixtures of appropriate solvents may be used, optionally containing water. For example:
- the fumarate salt is typically prepared using acetone, ethyl acetate, isopropanol or 2-butanol as the solvent, preferably isopropanol;
- the succinate salt is typically prepared using isopropanol or 2-butanol as the solvent, preferably isopropanol;
- the methane sulfonate salt is typically prepared using acetone, ethyl acetate, dimethylformamide, chloroform, methanol, ethanol, isopropanol or 2-butanol as the solvent, preferably methanol; and
- the saccharinate salt is typically prepared using methanol, ethanol, isopropanol or 2-butanol as the solvent, preferably methanol.

Upon completion of any of the foregoing reactions, the salt can be isolated from the reaction mixture by any conventional means such as precipitation, concentration, centrifugation and the like.

It will be appreciated that while specific process conditions (i.e. reaction temperatures, times, mole ratios of reactants, solvents, pressures, etc.) are given, other process conditions can also be used unless otherwise stated.

To prepare the salts of the present invention, the free base is typically dissolved in an appropriate solvent which is then heated to approximately 60-80°C. Then a solution of the appropriate dicarboxylic acid, sulfonic acid or sulfimide in an suitable solvent, preferably the same solvent as that in which the free base is dissolved, is typically added to the heated solution. The mixture is then optionally stirred for 60-120 minutes at 60-80°C. The mixture is then typically cooled, for example down to 20-25°C or 0-5 °C. The precipitate formed is isolated by filtration, washed with an appropriate solvent and dried for example in vacuum.

To prepare the fumarate salt of the present invention, the free base is preferably dissolved in isopropanol which is then heated to 70-80°C. Then a solution of fumaric acid dissolved in isopropanol is slowly added. The mixture is then stirred for 60-120 minutes at 70-80°C. The mixture is then typically cooled to 20-25°C. The precipitate formed is isolated by filtration, washed with isopropanol and dried.

To prepare the succinate salt of the present invention, the free base is preferably dissolved in isopropanol which is then heated to 70-80°C. Then a solution of succinic acid dissolved in isopropanol is slowly added. The mixture is then stirred for 60-120 minutes at 70-80°C. The mixture is then typically cooled to 20-25°C. The precipitate formed is isolated by filtration, washed with isopropanol and dried.

To prepare the methanesulfonate salt of the present invention, the free base is s preferably dissolved in methanol which is then heated to 60-70°C. Then a solution of methanesulfonic acid dissolved in methanol is slowly added. The mixture is cooled and kept at 1-5°C for 50 to 100 hours. The precipitate formed is isolated by filtration, washed with methanol and dried.

To prepare the saccharinate salt of the present invention, the free base is preferably dissolved in methanol which is then heated to 60-70°C. Then a solution of saccharin dissolved in methanol is slowly added. The mixture is cooled and kept at 1-5°C for 25 to 75 hours. The precipitate formed is isolated by filtration, washed with methanol. This s provides the hemimethanolate solvate form. Heating to 80 to 100 °C of the hemimethanolate solvate under vacuum for 4 to 6 hours removes the solvated methanol. The hemihydrate is typically formed by subsequent exposure to a humid atmosphere.

### Pharmaceutical compositions

The invention also encompasses pharmaceutical compositions comprising a therapeutically effective amount of a salt of the invention or an enantiomer or pharmaceutically acceptable solvate thereof and a pharmaceutically acceptable carrier.

Typically the pharmaceutical composition is formulated for administration by inhalation, preferably as a dry powder.

Typically, the pharmaceutical composition further comprises a therapeutically effective amount of one or more other therapeutic agents.

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Dry powder compositions for topical delivery to the lung by inhalation may, for example, be presented in capsules and cartridges of for example gelatine or blisters offer example laminated aluminium foil, for use in an inhaler or insufflator. Formulations generally comprise a powder mix for inhalation of the salt of the invention and a suitable powder base (carrier substance) such as lactose or starch. Use of lactose is preferred. The powder base may include additional components such as preservatives, stabilizing agents, absorption enhancers or aerodynamic modifier.

Each capsule or cartridge may generally contain between 0.1 µg and 150 µg of each therapeutically active ingredient. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring plungers with a local or circumferential recess to displace a certain volume of powder from the container to a delivery chamber or an air conduit (e. g. EP 0505321, WO 92/04068 and WO 92/04928), or measuring slides such as the Genuair® devise (formerly knows as Novolizer SD2FL) which is described in the following patent applications: WO 97/000703, WO 03/000325 and WO 03/061742.

### Additional therapeutic agents

The salts of the present invention may also be combined with one or more other therapeutic agents, in particular one or more drugs selected from the group consisting of corticosteroids, anticholinergic agents and PDE4 inhibitors. The invention is thus also directed to a combination comprising the salt of the invention with one or more other therapeutic agents known to be useful in the treatment of respiratory disorders, in particular one or more drugs selected from the group consisting of corticosteroids, anticholinergic agents and PDE4 inhibitors. The pharmaceutical compositions of the invention can also optionally comprise a therapeutically effective amount of one or more other therapeutic agents which are known to be useful in the treatment of respiratory disorders, such as PDE4 inhibitors, corticosteroids and/or anticholinergics.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day, most preferably once a day.

Examples of suitable PDE4 inhibitors that can be combined with β2-agonists are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, phenylethyl]pyridine(CDP-840),N-(3,5-Dichloro-4-pyridinyl)-2-[1-(4-fluorobenzyl)-5-hydroxy-1H-indol-3-yl]-2-oxoacetamide(GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4₋(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxvethyl)pyridin-2(1H)-one (T-440), (-)-trans-2-[3'-[3-(N-Cyctopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexanl-one, cis [4-cyano-4-(3-cyclopropylmethaxy-4-difluoromethoxyphenyl)cyclohexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number W003J097613, W02004/058729, WO 2005/049581, WO 2005/123693 and WO 2005/123692.

Examples of suitable corticosteroids and glucocorticoids that can be combined with β2-agonists are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR- 10654 1, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methyl sulfanyl)acetoxy] -4-pregnene-3,20-di one, Desisobutyrylciclesonide, hydrocortisone acetate, hydrocortisone sodium succinate, NS-126, prednisolone sodium phosphate and hydrocortisone probutate, Prednisolone sodium metasulfobenzoate and clobetasol propionate

Examples of suitable M3 antagonists (anticholinergics) that can be combined with β2-aganists are tiotropium salts, oxitropium salts, flutropium salts, ipratropium salts, glycopyrronium salts, trospium salts, zamifenacin, revatropate, espatropate, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,4-tetrahydroquinazoline-3-carboxylic acid enda-8-methyl-8-azabicyclo[3.2.1]act-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methy=lhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one(Banyu-280634),N-[N-[2-[N-[1-(Cyclohexylmethyl)piperidin-3(R)-ylmethyl]carbamayl]ethyl]carbamaylmethyl]-3, 3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3 (S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-Quorophenyl)-2-oxoimidazolidm-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azomatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Particularly preferred pharmaceutical compositions and combinations according to the invention comprise a salt of the invention and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO03/097613, W02004/058729, WO 2005/049581, WO 2005/123693 and WO 2005/123692.

Still particularly preferred pharmaceutical compositions and combinations according to the invention comprise a salt of the invention and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-{3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast and cilomilast

Thus, in one aspect of the invention, the composition or combination comprises a salt of the invention and a corticosteroid. Particularly preferred corticosteroids are those selected from the group consisting of mometasone furoate, ciclesonide, budesonide, fluticasone furoate and fluticasone propionate.

In another aspect of the invention, the composition or combination comprises a salt of the invention and an anticholinergic agent. Particularly preferred anticholinergic agents are those selected from the group consisting of tiotropium salts, glycopyrronium salts, 3-[2-Hydroxy-2,2-bis(2-thienyl)acetoxy]-1-(3-phenoxypropyl)-1-azoniabicycla[2.2.2]octane salts and 1-(2-Phenylethyl)-3-(9H-xanthen-9-ylcarbonyloxy)-1-azoniabicyclo[2.2.2]octane salts. The composition or combination may further comprise a corticosteroid selected from the group consisting of mometasone furoate, ciclesonide, budesonide, fluticasone furoate and fluticasone propionate.

In a still other aspect of the invention, the composition comprises a salt of the invention and a PDE4 inhibitor. Particularly preferred PDE4 inhibitors are those selected from the group consisting of rolipram, roflumilast, cilomilast and the compounds claimed in the PCT patent applications number WO03/097613, W02004/058729, WO 2005/049581, WO 2005/123693 and WO 2005/123692. The composition may further comprise a corticosteroid selected from the group consisting of mometasone furoate, ciclesonide, budesonide, fluticasone furoate and fluticasone propionate. In addition to the salt of the invention and to the PDE4 inhibitor, the composition or combination may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azonibicylo[2.2.2] octane salts and 1-(2-Phenylethyl)-3-(9H-xanthen-9-ylcarbonyloxy)-1-azoniabicyclo[2.2.2] octane salts.

In a particularly preferred embodiment of the present invention, the composition or combination comprises a salt of the invention and a therapeutically effective amount of a 3-[2-Hydraxy-2,2-bis(2-thienyl)aceroxy]-1-(3-phenoxypropyl)-1-azoniabicyclo[2.2.2]octane salt. Optionally, the composition or combination further comprises a corticosteroid and/or a PDE4 inhibitor.

In another particularly preferred embodiment of the present invention, the composition or combination comprises a salt of the invention and a therapeutically effective amount of mometasone furoate. Optionally, the composition further comprises an anticholinergic and/or a PDE4 inhibitor.

In yet another embodiment of the invention, the composition or combination comprises salt of the invention, a corticosteroid, an anticholinergic agent and a PDE4 inhibitor.

### Treatment of a pathological conditions or diseases associated with β2 adrenergic receptor activity

The salts of the invention, pharmaceutical compositions and the combinations of the invention may be used in the treatment of pathological conditions or diseases associated with β2 adrenergic receptor activity, typically respiratory diseases. The respiratory disease is preferably one in which the use of bronchodilating agents is expected to have a beneficial effect, for example asthma, acute or chronic bronchitis, emphysema, or Chronic Obstructive Pulmonary Disease (COPD). Asthma or chronic obstructive pulmonary disease are more preferred.

The active compounds and the salts in the combination, i.e. the β2-agonist of the invention and the PDE4 inhibitors, corticosteroids or glucocorticoids and/or anticholinergics may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially nebulisers and metered dose inhalers; however, any other form of topical, parenteral or oral application is possible. Here, the application of inhaled compositions embodies the preferred application form, especially in the therapy of obstructive lung diseases or for the treatment of asthma.

The active compound(s) formulations generally contain a suitable carrier which may be either a propellant for MDT administration or water for administration through a nebuliser. The formulation may comprise additional components such as preservatives (for example, benzalkonium chloride, potassium sorbate, benzyl alcohol); pH stabilizers (for example, acidic agents, alkaline agents, buffer systems); isotonic stabilizers (for example, sodium chloride); surfactant and wetting agents (for example, polysorbates, sorbitan esters); and/or absorption enhancers (for example, chitosan, hyaluronic acid, surfactants). The formulation may also contain additives to improve the solubility of other active compounds when mixed with the salt of the invention. The solubility enhancers may comprise components such as cyclodextrins, liposomes or co-solvents such as ethanol, glycerol and propylene glycol.

Additional suitable carriers for formulations of the active salts of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

The invention further encompasses a method of treating diseases or conditions associated with β2 adrenergic receptor activity, typically respiratory diseases, such as asthma or chronic obstructive pulmonary disease, in a mammal, the method comprising administering to the mammal, a therapeutically effective amount of a salt, pharmaceutical composition or combination or the invention. The mammal is preferably a human being.

### Examples

Reagents, starting materials, and solvents were purchased from commercial suppliers and used as received.

The FTIR spectra were recorded using Bruker Tensor 27, equipped with a MKII golden gate single reflection ATR System, a mid-infrared source as the excitation source and a DTGS detector. The spectra were acquired in 32 scans at a resolution of 4 cm⁻¹. No Sample preparation were required to perform the analysis.

DSC analyses were recorded in a Mettle Toledo DSC822e. Samples of 1-2 mg were weighted into 40 µL aluminium crucibles with a pinhole lid, and were heated, under Nitrogen (50 mL/min.), from 30 to 250°C at a heating rate between 2 and 20°C/min depending on the experiment. Data collection and evaluation was done with software STARe.

Thermogravimetric analyses were recorded in a Mettler Toledo S TA851e. Samples of 3-4 mg were weighted (using a microscale MX. 5, Mettler) into open 40 µL aluminium crucibles with a pinhole lid, and were heated, under Nitrogen (80 mL/min.), from 30 to 300°C at a heating rate of 10°C/min Data collection and evaluation was done with software STARe.

Proton nuclear magnetic resonance analyses were recorded in deuterate chloroform (CDC13) in a Bruker Avance 400 Ultrashield NMR spectrometer, equipped with a z-gradient 5 mm BBO (BroadBand Observe) probe with ATM and an automatic BACS-120 autosampler. Spectra were acquired solving 2-10 mg of sample in 0.6 mL of deuterated solvent.

In order to acquire a powder diffraction pattern of the obtained solid, approximately 20 mg of the non manipulated samples were prepared in standard sample holders using foils of poly acetate.

Powder diffraction pattern were acquired on a 8 Advance Series 2theta/theta powder diffraction system using Cu_{kα}-radiation in transmission geometry. The system is equipped with a VANTEC-1 single photon counting PSD, a Germanium monochromator a ninety positions auto changer sample stage, fixed divergence slits and radial soller: Programs used: data collection with DIFFRAC plus XRD Commander V.2.5.1 and evaluation with EVA V.12.0.

### Example 1 - preparation of 5-[(1R)-2-({2-[4-(2,2-difluoro-2-shenyl] ethyl}amino)-1-hysroxyethyl]-8-hydroxyquinolin-2(1H)-one fumarate

A solution of fumaric acid (119 mg, 1.03 mmols, 0.5 equivalents) in 5 mL of isopropanol was added slowly to a solution of 5-[(1R)-2-({2-[4-{2,2-difluoro-2-phenylethoxy)phenyl] ethyl}amino)-1-hydroxyethyl]-8-hydroxyquinolin-2(1H)-one (0.982 g, 2.04 mmols) in 35 mL of isopropanol at 75°C. During the addition a white precipitate was formed. The resulting suspension was stirred at 75°C for 90 minutes and cooled slowly to room temperature. The white solid was filtered off, washed with 10 mL of isopropanol and dried under vacuum (10 mm Hg) at 65°C for 5 hours to give the fumarate salt as a white solid (0.934 g, yield 85%).

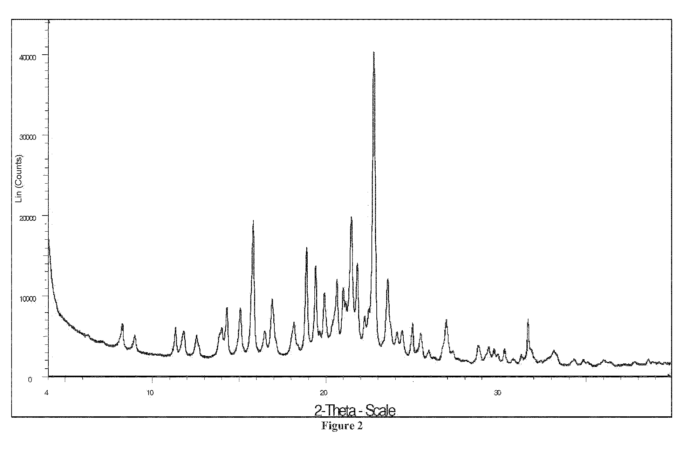

Figure 2 shows the powder X-ray diffraction (PXRD) pattern for the fumarate salt. A large number of peaks were observed in the whole range of 2θ measured, which corresponds to a very crystalline sample.

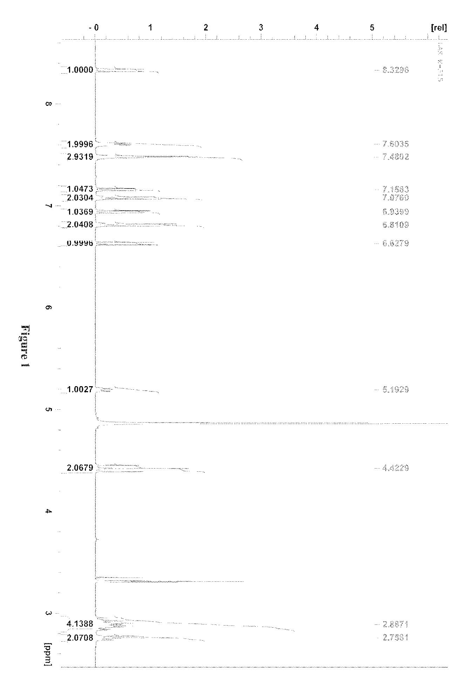

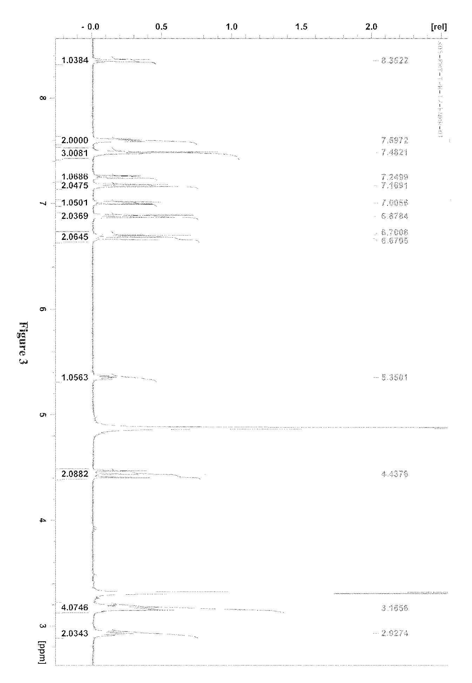

Figure 3 shows the ¹H-NMR (400 MHz, *d4*-methanol) for the fumarate salt. The expected variation on the chemical shifts from the free base (Figure 1) to the salt is observed. The appearance of the signal corresponding to the hydrogen atoms of the fumarate and its integration confirms the formation of the fumarate salt. Signals appeared at δ: 2.89 - 2.97 (m, 2H), 3.12 - 3.22 (m, 4H), 4.44 (t, *J* = 12 Hz, 2H), 5.3 5 (dd, *J* = 5 Hz, *J* = 8 Hz, 1H), 6.67 (d, *J* = 10 Hz, 1H), 6.70 (s, 1H), 6.88 (d, *J* = 9 Hz, 2H), 7.00 (d, *J* = 8 Hz, 1H), 7.17 (d, *J* = 9 Hz, 2H), 7.25 (d, *J* = 8 Hz, 1H), 7.44 - 7.52 (m, 3H), 7.56 - 7.63 (m, 2H), 8.3 (d, *J* =1 10 Hz, 1H).

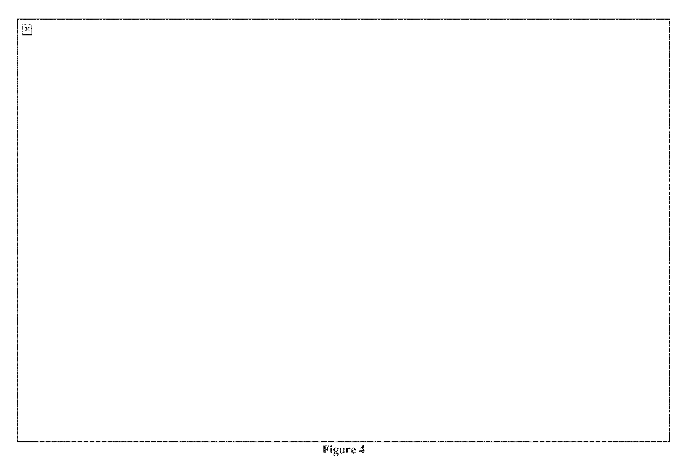

Figure 4 shows the differential scanning calorimetry (DSC) analysis of the fumarate salt. With a heating rate of 10°C/min, an endothermic intense peak at 177°C is observed. When the gradient temperature is changed, the endothermic peak shifts: down to 170°C at a rate of 2°C/min and up to 187°C at a rate of 20°C/min. Additionally, the visual observation on the microscope hot stage shows a change of colour from white to yellow associated with a melting of crystals in the same temperature range.

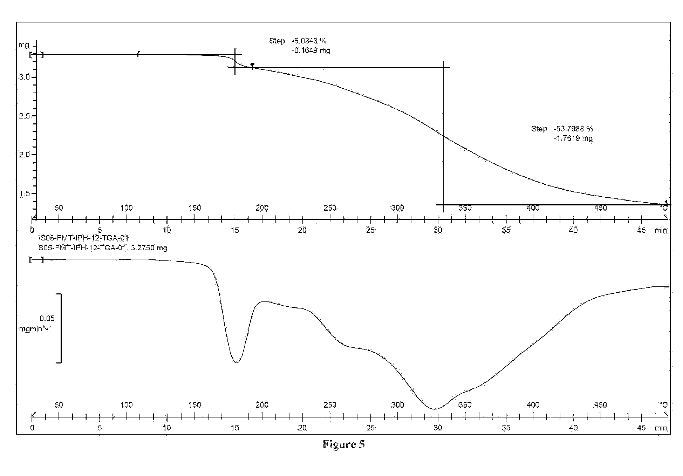

Figure 5 shows the thermogravimetric (TG) analysis of the fumarate salt. Weight loss at temperatures higher than 175°C was observed, which coincides with the temperature range of the endothermic peak in the DSC. This indicates that the endothermic peak observed in the DSC corresponds to an endothermic decomposition and not to the melting point.

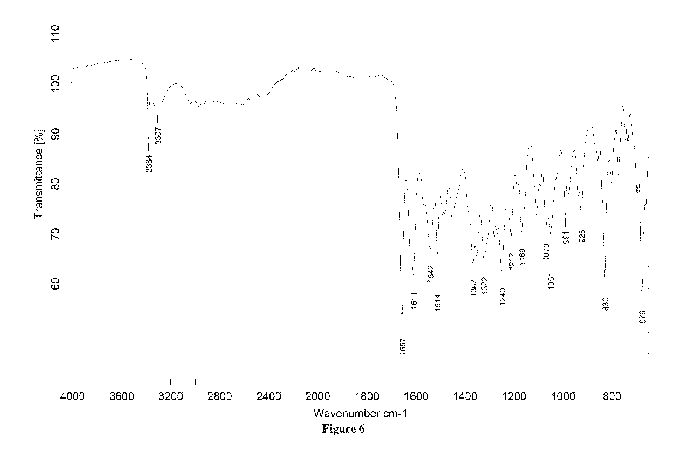

Figure 6 shows the Fourier transform infrared (FTIR) spectrum (ATR) for the fumarate salt. Signals appeared at υ: 3384, 3307, 1657, 1611, 1542, 1514, 1367, 1322, 1249, 1212, 1169, 1070, 1051, 991, 926, 830 and 679 cm⁻¹.

### Example 2 - preparation of 5-[(1R)-2-({2-[4-(2.2-difluoro-2-phenylethoxy)phenyl] ethyl)amino)-1-hydroxyefhl]-8-hydroxyquinolin-2(1H)-one succinate

A solution of succinic acid (140 mg, 1.186 mmols, 0.5 equivalents) in 5 mL of isopropanol was added slowly to a solution of 5-[(1R)-2-({2-[4-(2,2-difluoro-2-phenylethoxy)phenyl] ethyl}amino)-1-hydroxyethyl]-8-hydroxyquinolin-2(1H)-one (1.146 g, 2.385 mmols) in 35 mL of isopropanol at 75°C. During the addition a yellowish precipitate was formed. The resulting suspension was stirred at 75°C for 2 hours and cooled slowly to room temperature. The solid was filtered off, washed with 6 mL of isopropanol and dried under vacuum (10 mm Hg) at 65°C for 5 hours to give the succinate salt as a white solid (1.02 g, yield 79%).

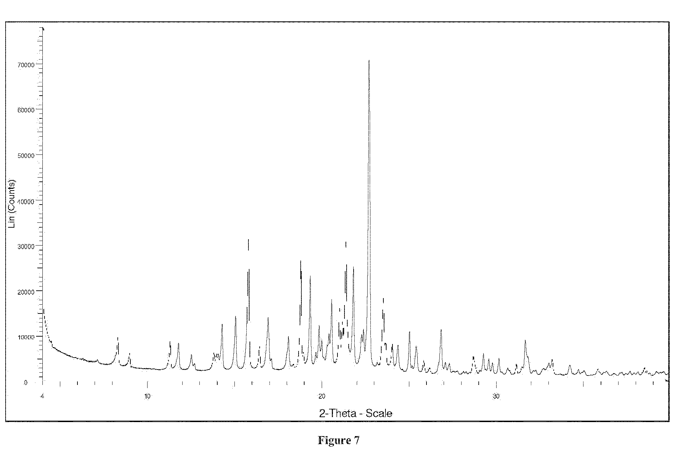

Figure 7 shows the PXRD analysis of the succinate salt. A large number of peaks were observed in the whole range of 2θ measured, which corresponds to a very crystalline sample. In addition, the succinate salt has the same diffractogram pattern as the fumarate salt, indicating that both salts are isomorphs, caused by the high similarity in the structures of fumarate and succinate. This fact confirms the formation of succinate and not hydrogensuccinate salt.

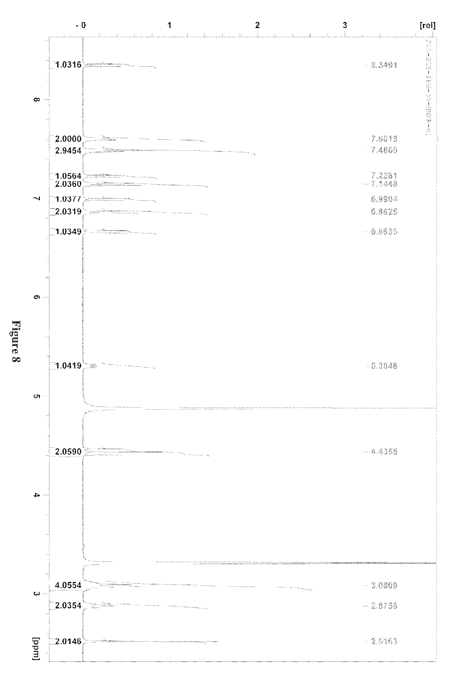

Figure 8 is the ¹H-NMR spectrum of the succinate salt. The expected variation on the chemical shifts from the free base (Figure 1) to the salt is observed. The appearance of the signal corresponding to the hydrogen atoms of the succinate and its integration confirms the formation of the succinate salt. ¹H-NNM (400 MHz, *d4*-methanal) δ: 2.52 (s, 2H), 2.84 - 2.92 (m, 2H), 3.03 - 3.14 (m, 4H), 4.43 (t, *J*= 12 Hz, 2H), 5.30 (dd, *J* = 5 Hz, *J* = 8 Hz, 1H), 6.66 (d, *J* = 10 Hz, 1H), 6.86 (d, *J* = 9 Hz, 2H), 6.99 (d, *J* = 8 Hz, 1H), 7.14 (d, J = 8 Hz, 2H), 7.23 (d, *J* = 8 8 Hz, 1H), 7.45 - 7.52 (m, 3H), 7.57 - 7.63 (m, 2H), 8.35 (d, *J* = 10 Hz, 1H).

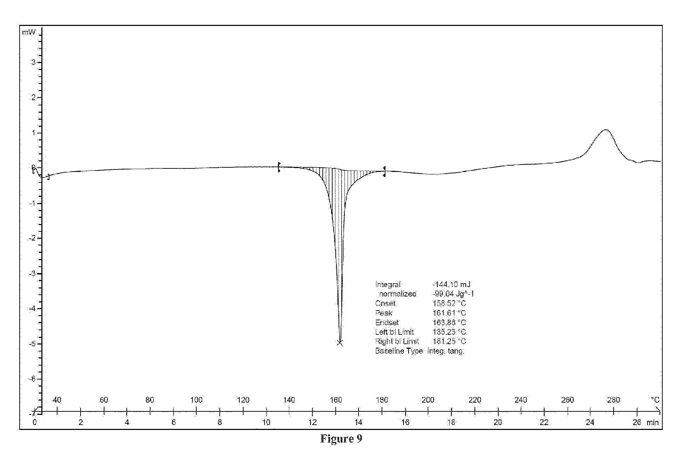

Figure 9 shows the DSC analysis of the succinate salt. With a heating rate of 10°C/min, an endothermic intense peak at 160°C was observed. The visual observation on the microscope hot stage shows a change of colour from white to yellow associated with a melting of crystals in the same temperature range.

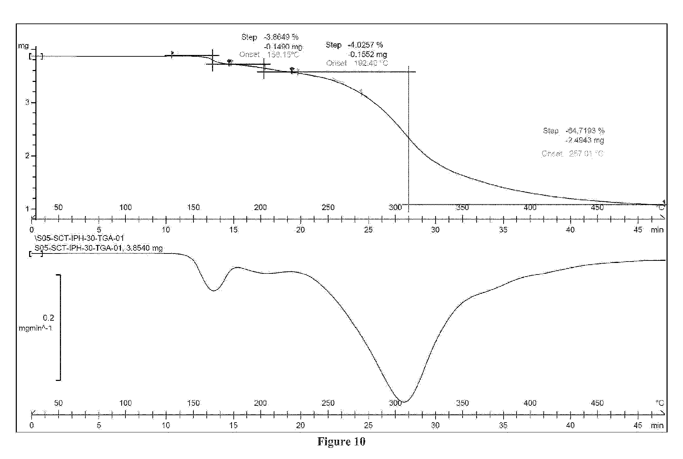

Figure 10 shows the TG analysis of the succinate salt. A weight loss at temperatures higher than 159°C was observed, which coincides with the temperature range of the endothermic peak in the DSC. These two observations indicate that the endothermic peak observed in the DSC corresponds to an endothermic decomposition and not to the melting point.

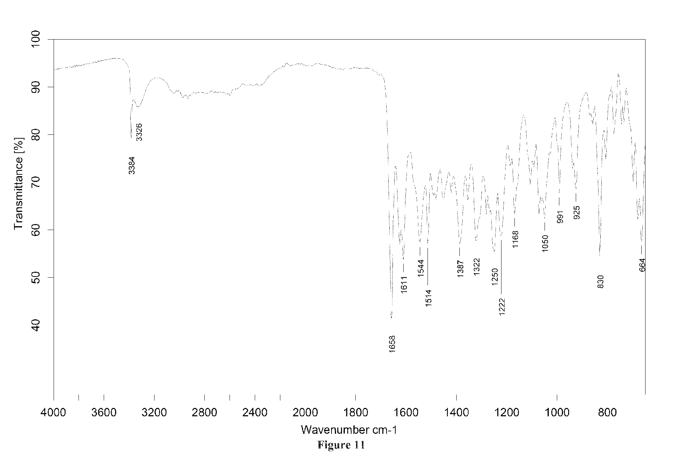

Figure 11 shows the FTIR spectrum (ATR) for the succinate salt. Signals appeared at υ: 3384, 3326, 1658, 1611, 1544, 1514, 1387, 1322, 1250, 1222, 1168, 1050, 991, 925, 830 and 664 cm⁻¹_{.}

### Example 3 - preparation of 5-[(1R)-2-({2-[4-(2,2-difluoro-2-phenylethoxy)phenyl] ethyl}amino)-1-hydroxyethyl]-8-hydroxyquinolin-2(1H)-one methanesulfonate

A solution of methanesulfonic acid (72 µL, 1.12 mmols, 1 equivalent) in 1 mL of methanol was added slowly to a solution of 5-[(1R)-2-({2-[4-{2,2-difluoro-2-phenylethoxy)phenyl] ethyl}amino)-1-hydroxyethyl]-8-hydroxyquinolin-2(1H)-one (532 mg, 1.107 mmols) in 5 mL of methanol at 65°C. The resulting solution was cooled to room temperature and kept for 72 hours at 4°C. The white solid formed was filtered off, washed with 3 mL of cold methanol and dried under vacuum (10 mm Hg) at 50°C for 5 hours to give the methanesulfonate salt as a white solid (385 mg, yield 60%).

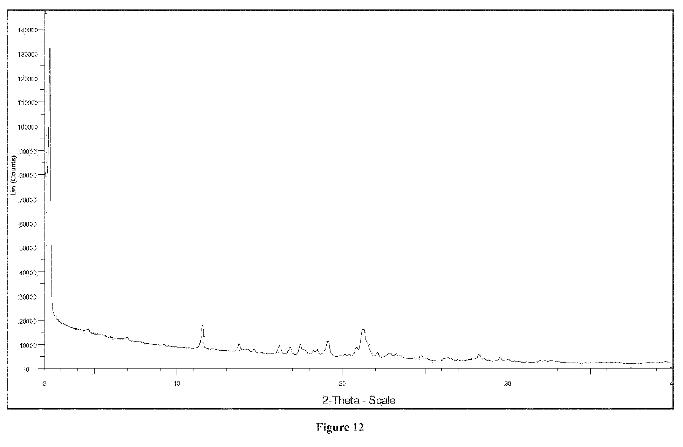

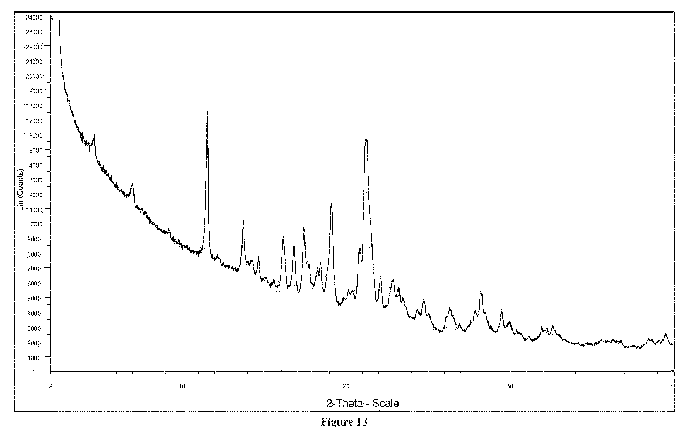

Figures 12 and 13 show the PXRD analysis of the methanesulfonate salt. An intense diffraction peak was observed at low angles, followed by many peaks in the whole range of 2θ with a lower intensity

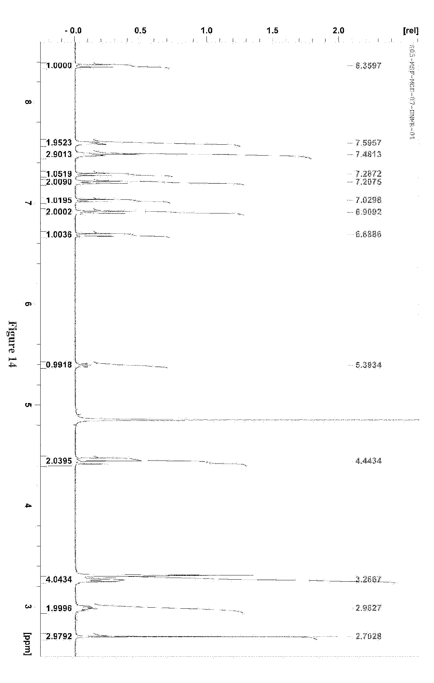

Figure 14 is the ¹H-NMR spectrum of the methanesulfonate salt. The expected variation on the chemical shifts from the free base (Figure 1) to the salt is observed. The appearance of the signal corresponding to the hydrogen atoms of the methyl group of methanesulfonate and its integration confirms the formation of the salt. ¹H-NMR (400 MHz, *d4*-methanal) δ: 2.70 (s, 3H), 2.94 - 3.02 (m, 2H), 3.23 - 3.30 (m, 4H), 4.44 (t, *J =* 12 Hz, 2H), 5.39 (dd, *J* = 5 Hz, *J* = 8 Hz, 1H), 6.69 (d, *J* = 10 Hz, 1H), 6.91 (d, *J* = 9 Hz, 2H), 7.03 (d, *J=* 8 Hz, 1H), 7.21 (d, *J* = 9 Hz, 2H), 7.29 (d, *J* = 8 Hz, 1H), 7.45 - 7.51 (m, 3H), 7.57 - 7.62 (m, 2H), 8.36 (d, *J* = 10 Hz, 1H).

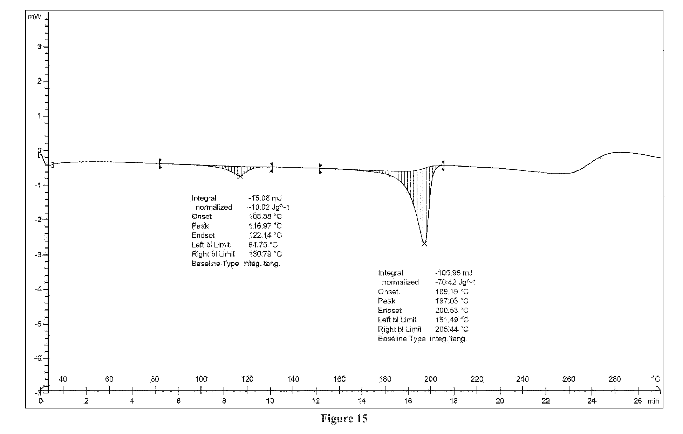

Figure 15 shows the DSC analysis of the methanesulfonate salt. Two endothermic peaks were observed. At a rate of 10°Clmin, a weak and broad peak with a maximum at 118ºC and a mid-intense broad peak starting at 189°C appear. The visual observation on the microscope hot stage does not show any change on the temperature range of the first band, but a change of colour from white to brown associated with a melting of crystals is observed over 180°C.

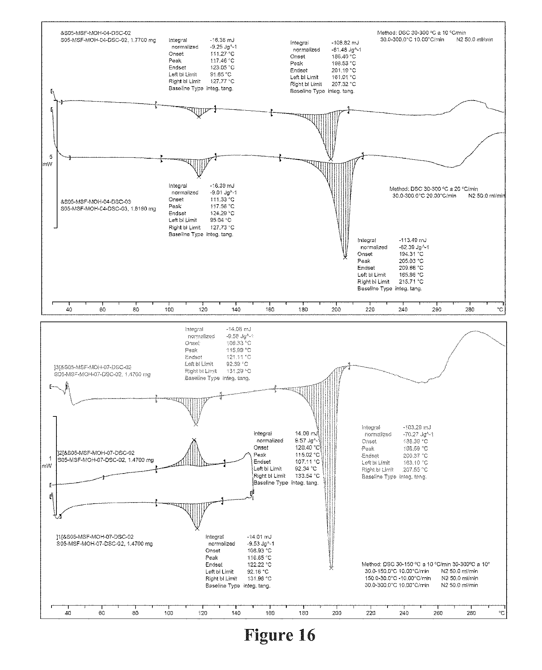

Further experiments on the DSC analysis revealed that the lower endothermic peak did not shift when the heating rate was changed and, on the other hand, the second peak shifted to a higher temperature when the heating rate was increased. A comparing DSC at two heating rates is shown in Figure 16 left. The first peak is reversible, as is shown in a DSC experiment when heating the salt to 150°C, cooling it to room temperature and reheating to 300°C as shown in Figure 16 right). As expected, on heating the peak is endothermic and on cooling exothermic. Since the position of the peak does not change in the heating or cooling processes, this transition has very little hysteresis. This fact indicates that this process has a fast kinetics and the temperature where these peaks appear in the DSC is very close to the thermodynamic transition temperature. With these observations it can be concluded that the first endothermic peak observed in the DSC corresponds to a polymorphic reversible transition and the second peak to an endothermic decomposition and not to the melting point.

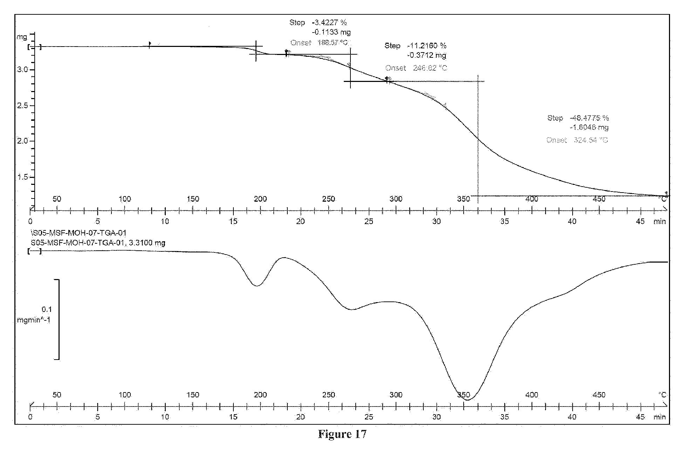

Figure 17 shows the TG analysis of the methanesulfonate salt. A weight loss at temperatures higher than 189°C was observed, which coincides with the temperature range of the second endothermic band in the DSC. No weight loss is observed in the temperature range of the first peak in the DSC.

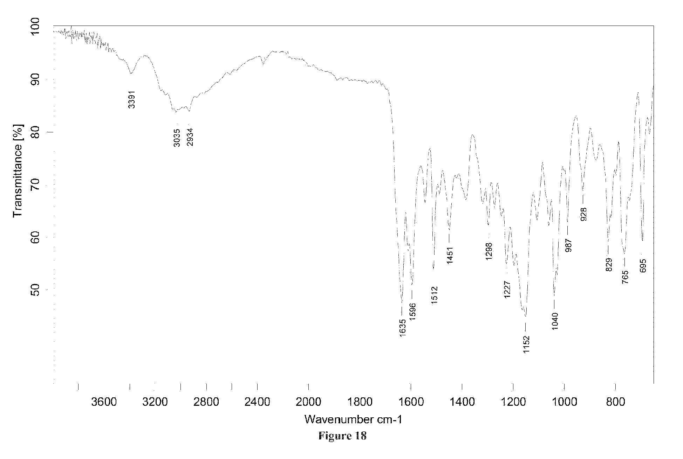

Figure 18 shows the FTIR (ATR) spectrum for the methanesulfonate salt. Signals appeared at υ: 3391, 3035, 2934, 1635, 1596, 1512, 1451, 1298, 1227, 1152, 1040, 987, 928, 829, 765 and 695 cm⁻¹.

### Example 4 - preparation of 5-[(1R)-2-({2-[4-(2,2-difluoro-2-phenylethoxy)phenyl] ethyl}amino)-1-hydroxyethyl]-8-hydroxyqquinolin-2(IH)-one saccharinate

A solution of saccharin (197 mg, 1.074 mmols, 1 equivalent) in 2 mL of methanol was added slowly to a solution of 5-[(1R)-2-({2-[4-(2,2-difluoro-2-phenylethoxy)phenyl] ethyl}amino)-1-hydroxyethyl]-8-hydroxyquinotin-2(1H)-one (516 mg, 1.074 mmols) in 3 mL of methanol at 65°C. The resulting solution was cooled to room temperature, about half of the solvent was allowed to evaporate, and the resulting solution was kept for 48 hours at 4°C. The white solid formed was filtered off, washed with 1 mL of cold methanol and dried under vacuum (10 mm Hg) at 50°C for 5 hours to give the saccharinate salt in form of a hemimethanolate as a white solid (500 mg, yield 67%).

The hemimethanolate obtained was heated under vacuum at 90°C for 5 hours to remove the solvent. Afterwards the solid was kept for 24 hours in a humid atmosphere and 2 days open to the air. This process gave the saccharinate salt in form of a hemihydrate as a white solid.

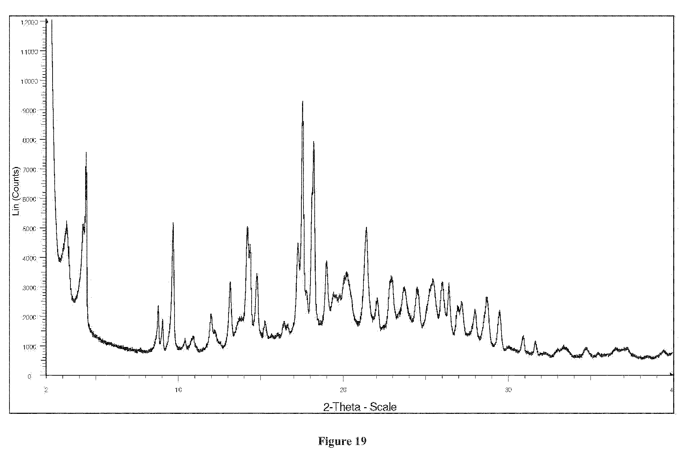

Figure 19 shows the PXRD analysis of the saccharinate salt hemihydrate. There are a large number of peaks situated in the whole range of 2θ measured, which corresponds to a very crystalline sample.

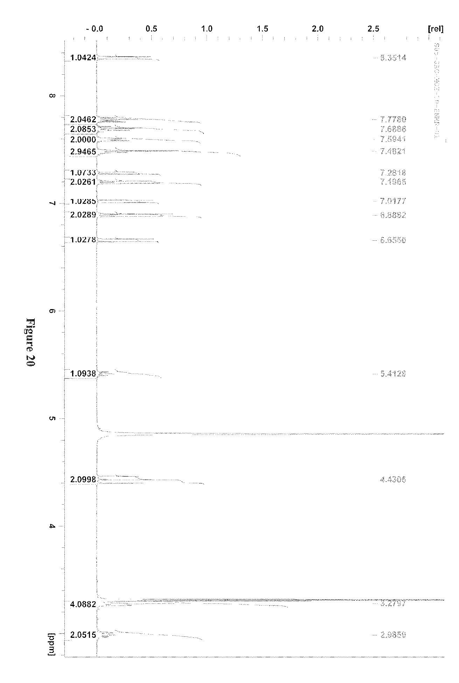

Figure 20 is the ¹H-NMR spectrum of the saccharinate salt hemihydrate. On the 1H-NMR spectrum the usual variation on the chemical shifts from the free base (Figure 1) to the salt is observed. The appearance of the signals corresponding to the hydrogen atoms of saccharine and its integration confirms the formation of the salt. 1H-NMR (400 MHz, d4-methanol) δ: 2.94 - 3.03 (m, 2H), 3.24 - 3.30 (m, 4H), 4.43 (t, J = 12 Hz, 2H), 5.41 (t, J = 7 Hz, 1H), 6.66 (d, J = 10 Hz, 1H), 6.89 (d, J = 9 Hz, 2H), 7.02 (d, J = 8 Hz, 1H), 7.20 (d, J = 9 Hz, 2H), 7.28 (d, J = 8 Hz, 1H), 7.44 - 7.51 (m, 3H), 7.56 - 7.62 (m, 2H), 7.65 - 7.72 (m, 2H), 7.73 - 7.81 (m, 2H), 8.35 (d, J = 10 Hz, 1H).

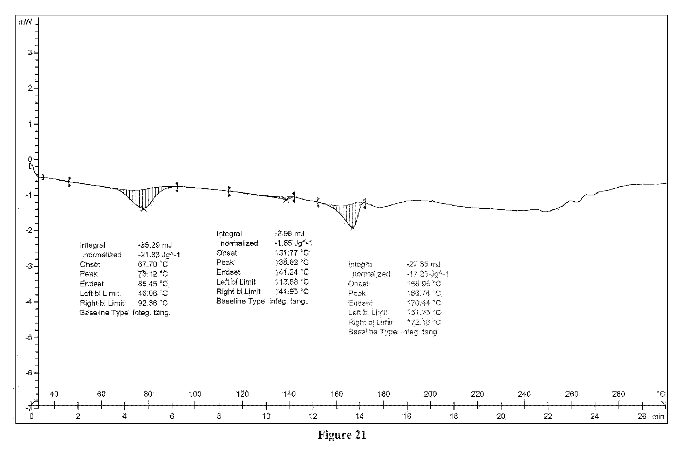

Figure 21 shows the DSC analysis of the saccharinate salt hemihydrate. Three endothermic peaks are observed. At a rate of 10°C/min, endothermic peaks with onsets at 68°C (weak, solvent evaporation), 132°C and 159°C (endothermic decomposition) were observed. On the DSC analysis (Figure 31) three endothermic peaks are observed. The visual observation on the microscope hot stage does not show any change around the first and second peaks, but shows a change of colour from white to brown associated with a melting of crystals around 160 - 170°C. The first peak corresponds to the loss of solvent.

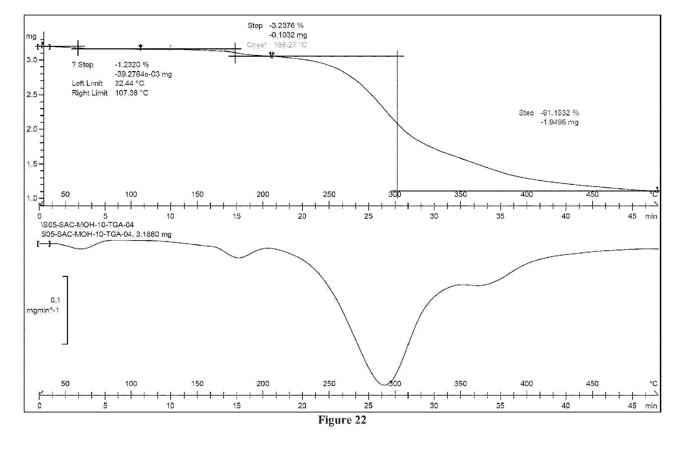

Figure 22 shows the TG analysis of the saccharinate salt hemihydrate. There is a weight loss at temperatures higher than 168°C, which coincides with the third endothermic peak on the DSC.

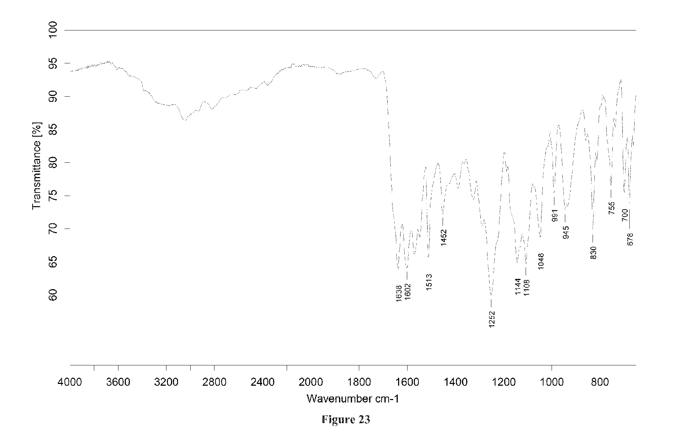

Figure 23 shows the FTIR spectrum (ATR) for the saccharinate salt hemihydrate. Signals appeared at υ: 3043, 1638, 1608, 1570, 1513, 1452, 1252, 1144, 1108, 1048, 991, 945, 830, 755, 700 and 678 cm-1.

## Claims

1. A pharmaceutically acceptable crystalline addition salt of (i) 5-[(1R)-2-({2-[4-(2,2-difluoro-2-phenylethoxy)phenyl]ethyl}amino)-1-hydroxyethyl]-8-hydroxyquinalin-2{1H)-one, and (ii) a dicarboxylic acid, a sulfonic acid or a sulfimide, or a pharmaceutically acceptable solvate thereof.

2. A salt according to claim 1, in which (ii) is a dicarboxylic acid.

3. A salt according to claim 2, which is:
5-[(1R)-2-({2-[4-(2,2-difluoro-2-phenylethoxy)phenyl]ethyl}amino)-1-hydroxyethyl]-8-hydraxyquinalin-2(1H)-ane fumarate; or
5-[(1R)-2-({2-[4-(2,2-difluoro-2-phenylethoxy)phenyl]ethyl] amino)-1-hydroxyethyl]-8-hydroxyquinolin-2(1H)-one succinate,
or pharmaceutically acceptable solvate thereof.

4. A salt according to claim 3, which is 5-[(1R)-2-({2-[4-(2,2-difluoro-2-phenylethoxy)phenyl]ethyl}amino)-1-hydroxyethyl]-8-hydroxyquinolin-2(1H)-one fumarate or pharmaceutically acceptable solvate thereof.

5. A salt according to claim 1, in which (ii) is a sulfonic acid or a sulfimide.

6. A salt according to claim 5, which is:
5-[(1R)-2-({2-[4-(2,2-difluoro-2-phenylethoxy)phenyl]ethyl}amino)-1-hydroxyethyl]-8-hydroxyquinolin-2(1H)-ane methanesulphonate; or
5-[(1R)-2-({2-[4-(2,2-difluoro-2-phenylethoxy)phenyl]ethyl}amino)-1-hydroxyethyl]-8-hydroxyquinolin-2(1H)-one saccharinate,
or pharmaceutically acceptable solvate thereof.

7. A pharmaceutical composition comprising a therapeutically effective amount of a salt as defined in any one of the preceding claims and a pharmaceutically acceptable carrier.

8. A pharmaceutical composition according to claim 7, which is formulated for administration by inhalation as a dry powder.

9. A pharmaceutical composition according to claim 7 or 8, which further comprises a therapeutically effective amount of one or more other therapeutic agens.

10. A pharmaceutical composition according to claim 9 wherein the other therapeutic agent is a corticosteroid, an anticholinergic agent, and/or a PDE4 inhibitor.

11. A pharmaceutical composition according to claim 9 or 10 wherein the other therapeutic agent is a corticosteroid selected from the group consisting of prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciclesonide, desisobutyrylciclesonide, hydrocortisone acetate, hydrocortisone sodium succinate, NS-126, prednisolone sodium phosphate, hydrocortisone probutate, prednisolone sodium metasulfobenzoate and clobetasol propionate.

12. A pharmaceutical composition according to claim 9 or 10 wherein the other therapeutic agent is an anticholinergic agent selected from the group consisting of tiotropium salts, oxitropium salts, flutropium salts, ipratropium salts, glycopyrronium salts, trospium salts, zamifenacin, revatropate, espatropate, NPC-14695, BEA-2108, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbanylaxy)-1-azoniabicycla[2.2.2]octane salts, 2-oxo-1,2,3,4-tetrahydroquinazoline-3-carboxylic acid endo-8-methyl-8-azabicycla[3.2.1]act-3-yl ester salts (DAU-5884), 3-(4-Benzylpiperazin-1-yl)-1-cyclobutyl-l-hydroxy-l-phenylpropan-2-one (NPC-14695), N-[1-(6-Aminapyridin-2-ylmethyl)piperidin-4-yl]-2(R)-[3,3-difluoro-1(R)-cyclopentyl]-2-hydroxy-2-phenylacetamide (J-104135), 2(R)-Cyclapentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3 -pentenyl)-4-piperidinyl]-2-phenylacetamide (J-104129), 1 -[4-(2-Aminoethyl)piperidin-l-yl]-2(R)-[3,3-diftuorocyclopent-1(R)-yl]-2-hydroxy-2-phenylethan-1-one(Banyu-280634),N-[N-[2-[N-[1-(Cyclohexylmethyl)piperidin-3(R)-ylmethyl]carbamoyl]ethyl]carbamoylmethyl]-3,3,3-triphenylprapionamide (Banyu CPTP), 2(R)-Cyclopentyl-2-hydroxy-2-phenylaceti acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1 -methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyt]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, , 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetaxy]-1-methyl-1-(2-phenoxyethyl) piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azaniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts.

13. A pharmaceutical composition according to claim 9 or 10 wherein the other therapeutic agent is a PDE4 inhibitor selected from the group consisting of benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840),N-(3,5-Dichloro-4-pyndinyt)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide(D-4418),N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsutfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one(T-440), (-)-trans-2-[3'-[3-(N-Cyclopropylcarbamoyl)-4-oxo-1,4-dihydro-1,8-naphthyridin-1-yl]-3-fluarobiphenyl-4-yl]cycloprapanecarboxylic acid (MK-0873), CDC-801, UK-500001, BLX-914,, 2-carbamethoxy-4-cyano-4-(3-cyclopropylmethoxy-4-difluroromethoxyphenyl)-cyclohexanl-one, *cis* [4-cyano-4-(3-cyclopropylmethoxy-4-difluaramethoxyphenyl)-cyclahexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the compounds claimed in the PCT patent applications number WO03/097613, WO2004/058729, WO 2005/049581, WO 2005/123693 and WO 2005/123692.

14. A pharmaceutical composition according to claim 9 or 10 wherein the other therapeutic agent is selected from the group consisting of mometasone furoate, ciclesonide, budesonide, fluticasone propionate, fluticasone furoate, tiotropium salts, glycopirrolium salts, 3-[2-Hydroxy-2,2-bis(2-thienyl)acetoxy]-1-(3-phenoxypropyl)-1-azaniabicyclo[2.2.2]octane salts, 1-(2-Phenylethyl)-3-(9H-xanthen-9-ylcarbonyloxy)-1-azoniabicyclo[2.2.2]octane salts, rolipram, roflumilast and cilomilast .

15. A combination comprising a salt as defined in any one of claims 1 to 6 and one or more other therapeutic agent, as defined in any one of claims 7 to 14.

16. A salt according as defined in any one of claims 1 to 6, a pharmaceutical composition as defined in any one of claims 7 to 14 or a combination as defined in claim 15, for use in the treatment of a pathological condition or disease associated with β2 adrenergic receptor activity.

17. A salt, pharmaceutical composition or combination for use as defined in claim 16, wherein the pathological condition or disease is asthma or chronic obstructive pulmonary disease.

18. Use of a salt as defined in any one of claim 1 to 6, a pharmaceutical composition as defined in any one of claims 7 to 14 or a combination as defined in claim 15, in the manufacture of a medicament for the treatment of a pathological condition or disease as defined in claim 16 or 17.

19. A method for treating a subject afflicted with a pathological condition or disease as defined in claim 16 or 17, which comprises administering to said subject an effective amount of a salt as defined in any one of claim 1 to 6, a pharmaceutical composition as defined in any one of claims 7 to 15 or a combination as defined in claim 15.
